# EUROPEAN PATENT SPECIFICATION

(11) **EP 4 088 829 B1**
(45) Date of publication and mention of the grant of the patent: **19.11.2025**
(21) Application number: 21893476.8
(22) Date of filing: 16.08.2021
(51) Int. Cl.: B05D 5/08, G01N 35/10, A61B 5/15, B05D 1/18, B05D 3/00, B05D 3/02, B05D 3/10, B05D 7/14, B05D 7/00, C09D 127/18, C25F 7/00, C25F 3/06, C23F 1/14

(54) **PREPARATION METHOD FOR WEAR-RESISTANT SUPER-HYDROPHOBIC COATING ON SURFACE OF SAMPLING NEEDLE**
VERFAHREN ZUR HERSTELLUNG EINER VERSCHLEISSFESTEN SUPERHYDROPHOBEN BESCHICHTUNG AUF EINER OBERFLÄCHE EINER ABTASTNADEL
PROCÉDÉ DE PRÉPARATION DE REVÊTEMENT SUPER-HYDROPHOBE RÉSISTANT À L'USURE SUR LA SURFACE D'UNE AIGUILLE D'ÉCHANTILLONNAGE

(30) Priority: 23.11.2020 CN 202011322952
(43) Date of publication of application: 16.11.2022
(73) Proprietor: Chongqing University, Shapingba Chongqing 400030 (CN)
(72) Inventor: SUN, Lidong, Chongqing 400030 (CN); ZHAO, Kaiqi, Chongqing 400030 (CN)
(74) Representative: Huang, Liwei
(86) International application number: PCT/CN2021/112809
(87) International publication number: WO 2022/105324

(56) References cited:
- WO-A1-2010/142550
- WO-A2-2012/167017
- CN-A- 101 566 549
- CN-A- 102 346 195
- CN-A- 102 346 195
- CN-A- 110 359 044
- CN-A- 110 359 044
- CN-A- 110 938 860
- CN-A- 112 547 462
- JP-A- 2014 140 642

## Description

### TECHNICAL FIELD

The present invention relates to a method for preparing a rub-resistant superhydrophobic coating on the surface of a sampling needle.

### BACKGROUND

Sampling needles are widely applied to the fields of biological detection, medical diagnosis equipment, scientific research detection instruments and the like. At present, sampling needles used by high-end medical diagnostic equipment and detection instruments in China seriously depend on import, the surface hydrophilicity and capillary acting force of the sampling needles can cause serious liquid adhesion on the surfaces of the sampling needles, which causes contamination to the next to-be-detected sample, reduces the sample detection precision, and is an internationally recognised bottleneck problem. At present, a more effect way is to construct a superhydrophobic coating on the surface of the sampling needle so as to reduce or even avoid residues of sample reagents and flushing fluid on the inner and outer surfaces and the end face of the sampling needle. The construction of the superhydrophobic coating generally needs two conditions, one condition is that the surface has a micro/nano rough structure, and the other condition is that the coating has low surface energy. For metal or alloy materials, the surface micro/nano rough structure can be realised through an electrochemical method. For example, a nano structure can be prepared on a rough surface of a micron structure through an electrochemical anodic oxidation method, and then the nano structure is modified by a low-surface-energy material, so that the superhydrophobic surface can be prepared. However, the mechanical property of the interface of the nano structure and the micron structure prepared by the electrochemical method is poor, and the problems of poor binding force and the like exist in practical application. For example, in the using process of the sampling needle, the outer surface and the end face of the sampling needle may rub against a silica gel bottle cap of a sample reagent bottle, the superhydrophobic performance of the outer surface and the end face can be reduced after multiple times of rubbing, and therefore the practical application of the sampling needle is difficult. Therefore, how to prepare the rub-resistant superhydrophobic coating on the surface of the sampling needle, inhibit liquid adhesion on the surface, reduce the contamination carrying rate, prolong the service life of the sampling needle and further improve the detection precision and efficiency of equipment is an urgent problem to be solved.
CN 102346195A discloses to a sample needle, wherein it mentions forming a stainless-steel tube with a 30° cone, polishing, and then spraying 0.05 mm Teflon. CN 110359044A discloses a method of preparing superhyperphobic film on steel substrate surface, wherein a solution (a modified superhydrophobic silica ethanol solution) is sprayed onto the surface of the steel substrate.
WO 2010142550A1 discloses a method for manufacturing a needle cannula, wherein electro polishing is used to remove material from the inside of the needle cannula.

### SUMMARY

The present invention aims to provide a method for preparing a rub-resistant superhydrophobic coating on the surface of a sampling needle according to claim 1, comprising the following steps:
1) cleaning and drying a sampling needle, a sealed cathode tube and a cathode wire;
2) coating the surface of the cathode wire with a high-molecular polymer solution and drying;
3) inserting the cathode wire into the cathode tube, fixing the cathode wire at the axis of the cathode tube as a cathode, inserting the sampling needle into the cathode tube, and inserting the cathode wire into the sampling needle;
4) connecting a nozzle at one end of the cathode tube to a dynamic solution supply device;
5) connecting the sampling needle to a positive pole of a power supply, and connecting the cathode wire and the cathode tube to a negative pole of the power supply; after power-on, carrying out electrochemical etching after the dynamic solution supply device injects an etching electrolyte into the cathode tube; thereby a micro/nanoscale rough structure is formed on the surface of the sampling needle;
6) cleaning and drying the sampling needle after electrochemical etching is completed;
7) inserting the sampling needle vertically into a polytetrafluoroethylene (PTFE) emulsion, and lifting the sampling needle after the operation is kept for a certain period of time; and
8) taking out and drying the sampling needle to obtain a sampling needle with a rub-resistant superhydrophobic coating on the surface. Preferred embodiments of the present invention are defined in the dependent claims.

Further, the subsequent treatment after the step 7 is as follows: step 9) carrying out heat treatment on the sampling needle.

Further, the material of the sampling needle is one of stainless steel materials such as ferritic stainless steel (such as 409 and 430), austenitic stainless steel (such as 316, 316L, 304 and 304L), and martensitic stainless steel (such as 410 and 420); the material of the cathode wire is one of conductive materials such as stainless steel wire, copper wire, iron wire, platinum wire and titanium wire.

Further, the material of the cathode tube is one of conductive materials such as a metal tube, an alloy tube and a graphite tube; the material of a seal of the cathode tube is consistent with the material of the cathode tube; the size of a central hole is kept consistent with the diameter of the cathode wire, two round holes with a diameter of 1.5 mm are formed along the central line at a position 3 mm away from the central hole to serve as solution inflow holes, and the cleaning time is 0.5-4 h.

Further, in the step 1), the sampling needle, the sealed cathode tube and the cathode wire are successively placed in detergent, alcohol and deionised water for ultrasonic oscillation cleaning separately, wherein the cleaning time is 0.5-4 h; then the sampling needle, the cathode tube and the cathode wire are blow-dried by using a nitrogen spray gun; and then the sampling needle, the cathode tube and the cathode wire are dried, wherein the drying temperature is 50-80 °C, and the drying time is 0.5-4 h.

Further, in the step 2), the dried cathode wire in the step 2) is used as a matrix, and the surface of the cathode wire is repeatedly coated with a high-molecular polymer solution; the solvent of the high-molecular polymer solution is one of N-methylpyrrolidone (NMP) and N,N-dimethylformamide (DMF), the solute of the high-molecular polymer solution is one of polymer materials such as polylactic acid (PLA), polyvinyl chloride (PVC) and polyvinylidene fluoride (PVDF), the concentration of the high-molecular polymer solution is 10-100 g/L, and the coating frequency is 2-30 times.

Further, in the step 3), the cathode wire is inserted into the cathode tube, the cathode wire is fixed at the axis of the cathode tube and serves as a cathode, the sampling needle serves as an anode and is vertically inserted into the cathode tube, the cathode wire is inserted into the sampling needle, and the relative positions of the cathode wire, the cathode tube and the sampling needle are fixed;
further, in the step 4), the cathode tube is vertically fixed, and a nozzle at the lower end of the cathode tube is connected to the dynamic solution supply device;
further, in the step 5), the etching electrolyte is conveyed by a peristaltic pump; the solvent of a solution for electrochemical etching is one or more of common solvents such as water, ethylene glycol and glycerol; the solute of the solution for electrochemical etching is one of halogen compounds such as sodium chloride, potassium chloride, calcium chloride, copper chloride, ferric chloride, sodium bromide, calcium bromide, potassium bromide, copper bromide and ferric bromide, and the concentration of the solution is 0.05-3 mol/L; the voltage of electrochemical etching is 5-40 V, and the time of electrochemical etching is 0.5-120 min.

Furthermore, in the step 7), the polytetrafluoroethylene (PTFE) emulsion is an emulsion formed by dispersing PTFE particles in water, the mass fraction of the polytetrafluoroethylene (PTFE) is 20-60%, the time of immersing the sampling needle into the emulsion is 2-60 s, and the lifting speed is 20-200 mm/min.

Further, the temperature of heat treatment is 360-380 °C, and the time of heat treatment is 0.5-3 h.

It is worth noting that according to the present invention, firstly, a micron-sized rough structure is constructed on the surface of the sampling needle by adopting a cathode-coated three-electrode dynamic coaxial etching method; then a polytetrafluoroethylene (PTFE) film is prepared on the substrate surface of the sampling needle with the micron rough structure by using a dip-coating film forming method; after heat treatment, the surface of the sampling needle is in a rolling superhydrophobic state. The superhydrophobic coating prepared on the sampling needle by the method has good rub resistance, not only can reduce the liquid adhesion phenomenon on the surface, reduce the contamination carrying rate of the surface and improve the detection precision of the sampling needle, but also can prolong the service life of the superhydrophobic sampling needle, and has good industrial application prospects.

The sampling needle (for medical examination) prepared by the method disclosed by the present invention has the following remarkable advantages and beneficial effects:
1, the contamination carrying rate of the sampling needle is reduced, and the sample detection precision is improved;
2, the applicability is wide, and there is no requirement on the size of the sampling needle;
3, the coating is good in rub resistance, and the service life of the sampling needle is prolonged.

### BRIEF DESCRIPTION OF THE DRAWINGS

Figure 1 is a schematic diagram of a sampling needle electrochemical etching device, wherein in the figure, 1. sampling needle; 2. sealed cathode tube (with a sealed end being punched); 3. cathode wire (coated with a high-molecular polymer film); 4. liquid conveying tube; 5. peristaltic pump; 6. etching electrolyte storage tank; and 7. direct-current power supply.
Figure 2 is a scanning electron micrograph of the outer surface of a sampling needle after electrochemical etching.
Figure 3 is a contact angle test chart of the outer surface of a sampling needle with a rub-resistant superhydrophobic coating.
Figure 4 is a contact angle test chart of the outer surface of a rub-resistant superhydrophobic sampling needle after the rub-resistant superhydrophobic sampling needle rubs against a silica gel bottle cap up and down for 100 cycles.

### DETAILED DESCRIPTION

The present invention will be further described below in conjunction with the embodiments, but it should not be understood that the scope of the above-mentioned subject matter of the present invention is only limited to the following embodiments.

### Embodiment 1

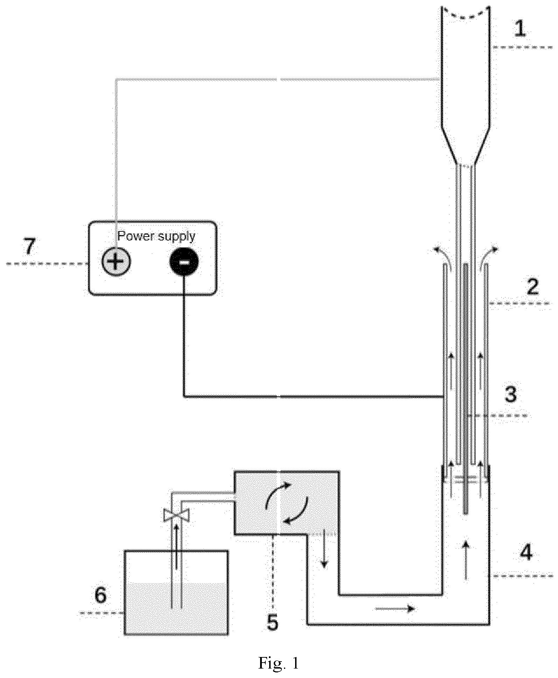

A device adopted in this embodiment is shown in Figure 1, in the figure, one end of a liquid conveying tube 4 is connected with a liquid outlet of a peristaltic pump 5, and the other end of the liquid conveying tube 4 is connected with an inlet at the lower end of a cathode tube 2. Liquid can be supplied into the cathode tube 2 by the peristaltic pump 5. An inlet of the peristaltic pump 5 is connected with an etching electrolyte storage tank 6 through a pipeline, and etching electrolyte can enter the peristaltic pump 5 through opening and closing of a valve and then be conveyed to the cathode tube 2 by the peristaltic pump 5. After being conveyed to the cathode tube 2, the etching electrolyte overflows from a nozzle at the upper end of the cathode tube 2 and/or the upper end of a sampling needle 1. A collecting tank for collecting overflowing liquid is arranged below the liquid conveying tube 4. A direct-current power supply 7 supplies power for the reaction.

In this embodiment,
a method for preparing a rub-resistant superhydrophobic coating on the surface of a sampling needle comprises the following steps:
1) placing a sampling needle, a sealed cathode tube and a cathode wire in detergent, alcohol and deionised water successively for ultrasonic oscillation cleaning separately, wherein the cleaning time is 0.5-4 h; then blow-drying the sampling needle, the cathode tube and the cathode wire by using a nitrogen spray gun; and then drying the sampling needle, the cathode tube and the cathode wire, wherein the drying temperature is 50-80 °C, and the drying time is 0.5-4 h;
2) coating the surface of the cathode wire with a high-molecular polymer solution and drying;
3) inserting the cathode wire into the cathode tube, fixing the cathode wire at the axis of the cathode tube as a cathode, inserting the sampling needle as an anode vertically into the cathode tube, inserting the cathode wire into the sampling needle, and ensuring that the sampling needle is both coaxial with the cathode tube and the cathode wire as much as possible;
4) inserting the cathode wire into the cathode tube, fixing the cathode wire at the axis of the cathode tube as a cathode, inserting the sampling needle as an anode vertically into the cathode tube, inserting the cathode wire into the sampling needle, and ensuring that the sampling needle is both coaxial with the cathode tube and the cathode wire, wherein the cathode wire is inserted into the cathode tube, the sampling needle is inserted into the cathode tube, the cathode wire is inserted into the sampling needle, and the relative positions of the cathode wire, the cathode tube and the sampling needle are fixed, so that it is ensured that the sampling needle is both coaxial with the cathode tube and the cathode wire as much as possible; connecting a nozzle at one end of the cathode tube to a dynamic solution supply device, wherein preferably, the material of the cathode tube is one of conductive materials such as a metal tube, an alloy tube and a graphite tube; the material of a seal of the cathode tube is consistent with the material of the cathode tube; a central hole of the nozzle at the upper end is used for allowing the cathode wire and the sampling needle to pass therethrough, the size of the hole is consistent with the diameter of the cathode wire, two round holes with the diameter of 1.5mm are formed along the central line at a position 3mm away from the central hole to serve as solution inflow holes, and in this embodiment, a nozzle at the lower end of the cathode tube is connected to the dynamic solution supply device;
5) connecting the sampling needle to a positive pole of a power supply, and connecting the cathode wire and the cathode tube to a negative pole of the power supply; after turn-on, turning on a switch of the peristaltic pump to introduce an etching electrolyte and control the flow velocity, and when the etching electrolyte flows out of the upper end of the cathode tube, connecting the power supply and carrying out electrochemical etching under a constant-voltage condition; thereby a micro/nanoscale rough structure is formed on the surface of the sampling needle;
6) cleaning and drying the sampling needle after electrochemical etching is completed;
7) inserting the sampling needle vertically into a polytetrafluoroethylene (PTFE) emulsion, and lifting the sampling needle after the operation is kept for a certain period of time; wherein the polytetrafluoroethylene (PTFE) emulsion is an emulsion formed by dispersing PTFE particles in water, the mass fraction of the polytetrafluoroethylene (PTFE) is 20-60%, the time of immersing the sampling needle into the emulsion is 2-60 s, and the lifting speed is 20-200 mm/min;
8) taking out and drying the sampling needle; and
9) carrying out heat treatment on the sampling needle to obtain a sampling needle with a rub-resistant superhydrophobic coating on the surface, wherein the temperature of heat treatment is 360-380 °C, and the time of heat treatment is 0.5-3 h.

### Embodiment 2

The main steps of this embodiment are the same as those of embodiment 1, further, in this embodiment,
the sampling needle (with the inner diameter of 0.4 mm, and the wall thickness of 0.2 mm, and made of 316L austenitic stainless steel), the stainless steel sealed cathode tube (with the inner diameter of 6 mm) (a hole with the diameter of 0.15 mm is punched in the centre of a seal) and the stainless steel cathode wire (with the diameter of 0.15 mm) in the step 1) are successively placed in detergent, alcohol and deionised water for ultrasonic oscillation cleaning separately, wherein the cleaning time is 2 h, the drying temperature is 50 °C, and the drying time is 1 h;
in the step 2), the dried cathode wire is used as a matrix, the surface of the cathode wire is repeatedly coated with a high-molecular polymer solution (with the solute being polyvinylidene fluoride and the solvent being N-methylpyrrolidone), the concentration of the solution is 10 g/L, and the coating frequency is 15-20 times; the drying temperature of the drying treatment is 60 °C, and the drying time is 1 h;
in the step 5), the outer surface of the sampling needle is connected to the positive pole of the power supply, the cathode wire and the cathode tube are connected to the negative pole of the power supply, the switch of the peristaltic pump is turned on to introduce the etching electrolyte (an aqueous solution of sodium chloride with the concentration of 0.5 mol/L) and control the flow speed to be 8 mL/min, when the etching electrolyte flows out of the upper end of the cathode tube, the power supply is connected, electrochemical etching is carried out under the constant-voltage condition, the voltage is 12 V, and the etching time is 3 min;
in the step 6), the obtained sampling needle is cleaned in deionised water and dried, the drying temperature is 60 °C, and the drying time is 2 h;
in the step 7), the obtained dry sampling needle is vertically inserted into a polytetrafluoroethylene (PTFE) emulsion with the mass concentration of 60 wt%, and is lifted after the operation is kept for 20 s, and the lifting speed is 200 mm/min;
in the step 8), the sampling needle is dried, the drying temperature is 70 °C, and the drying time is 0.5 h;
in the step 9), the sampling needle is subjected to heat treatment, during heat treatment, the heating temperature is 370 °C, the time of heat treatment is 1.5 h, and the temperature rise speed is 2.5 °C/min.

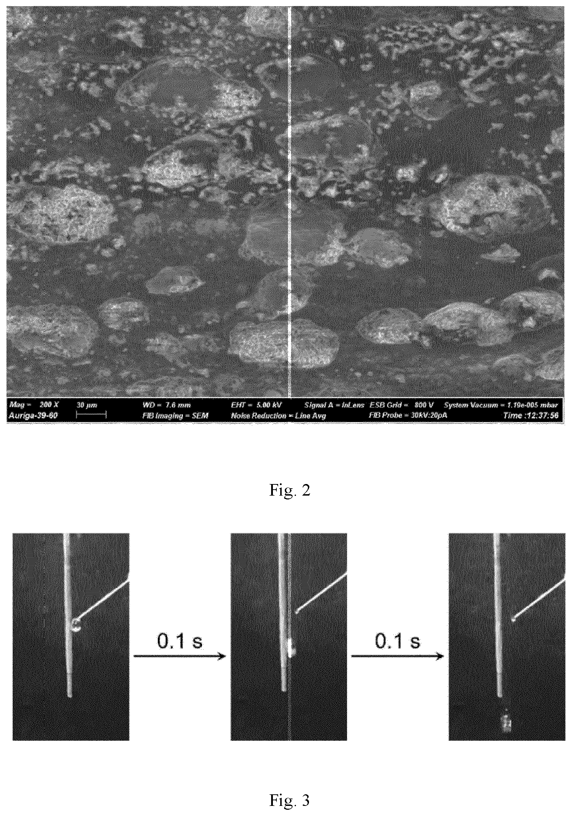

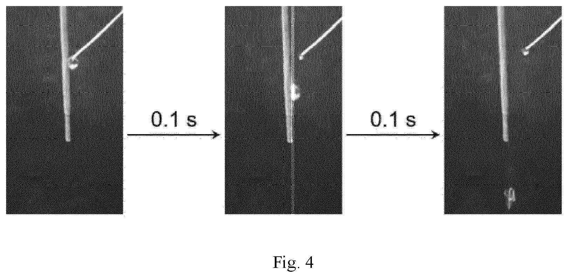

Relevant performance data of this embodiment is as follows:
the inner surface, the outer surface and the end face of the sampling needle subjected to electrochemical etching have certain micron-sized rough structures, which are shown in a scanning electron microscope morphology of Figure 2. A polytetrafluoroethylene (PTFE) film is prepared on the substrate surface of the sampling needle with the micron rough structure by a dip-coating film-forming method. The inner surface, the outer surface and the end face of the treated sampling needle are all in a rolling superhydrophobic state, when the sampling needle is in contact with water drops with the volume of about 6 µL, the liquid adhesion phenomenon does not occur, and the liquid drops can rapidly roll down along the outer surface of the sampling needle, as shown in a contact angle test chart of Figure 3. After the treated sampling needle rubs against a silica gel bottle cap perpendicular to the hole shaped like a Chinese character " 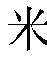 " up and down for 100 cycles, the outer surface of the sampling needle is still in the rolling superhydrophobic state, and the sampling needle has good rub resistance, as shown in a contact angle test chart of Figure 4.

### Embodiment 3

The difference from Embodiment 1 or 2 lies in that:
In the step 1), the inner diameter of the sampling needle is 0.8 mm, the material of the sampling needle is 304 austenitic stainless steel, the material of the sealed cathode tube is titanium alloy, and the material of the cathode wire is titanium wire.
In the step 2), the solute of the high-molecular polymer solution is polylactic acid (PLA), the solvent of the high-molecular polymer solution is N,N-dimethylformamide (DMF), the concentration of the solution is 30 g/L, and the coating frequency is 2-8 times.
In the step 5), the etching electrolyte is an ethylene glycol solution of sodium chloride with the concentration of 1 mol/L, the flow velocity of the etching electrolyte is 10 mL/min, the etching voltage is 5 V, and the etching time is 10 min.
In the step 7), the mass concentration of the polytetrafluoroethylene (PTFE) emulsion is 40wt%, and the lifting speed is 100 mm/min.
In the step 9), the temperature of heat treatment is 360 °C, and the time of heat treatment is 3 h.

### Embodiment 4

The difference from Embodiment 1 or 2 lies in that:
In the step 1), the inner diameter of the sampling needle is 1 mm, the material of the sampling needle is 316 austenitic stainless steel, the material of the sealed cathode tube is aluminum alloy, the inner diameter of the sealed tube is 8 mm, the material of the cathode wire is iron wire, and the diameter of the cathode wire is 0.2 mm.
In the step 2), the solute of the high-molecular polymer solution is polyvinyl chloride (PVC), the solvent of the high-molecular polymer solution is N-methylpyrrolidone (NMP), the concentration of the solution is 15 g/L, and the coating frequency is 5-10 times.
In the step 5), the etching electrolyte is an aqueous solution of sodium bromide with the concentration of 1.5 mol/L, the flow velocity of the etching electrolyte is 8 mL/min, the etching voltage is 25 V, and the etching time is 100 s.
In the step 7), the mass concentration of the polytetrafluoroethylene (PTFE) emulsion is 45wt%, and the lifting speed is 50 mm/min.
In the step 9), the temperature of heat treatment is 380 °C, and the time of heat treatment is 1 h.

### Embodiment 5

The difference from Embodiment 1 or 2 lies in that:
In the step 1), the inner diameter of the sampling needle is 1.2 mm, the material of the sampling needle is 304L austenitic stainless steel, the material of the sealed cathode tube is copper alloy, the inner diameter of the sealed tube is 10 mm, the material of the cathode wire is copper wire, and the diameter of the cathode wire is 0.3 mm.
In the step 2), the solute of the high-molecular polymer solution is polyvinyl chloride (PVC), the solvent of the high-molecular polymer solution is N,N-dimethylformamide (DMF), the concentration of the solution is 30 g/L, and the coating frequency is 5-8 times.
In the step 5), the etching electrolyte is an aqueous solution of calcium chloride with the concentration of 2 mol/L, the flow velocity of the etching electrolyte is 15 mL/min, the etching voltage is 30 V, and the etching time is 0.5 min.
In the step 7), the mass concentration of the polytetrafluoroethylene (PTFE) emulsion is 35wt%, and the lifting speed is 25 mm/min.

### Embodiment 6

The difference from Embodiment 1 or 2 lies in that:
In the step 1), the material of the sampling needle is 410 martensitic stainless steel, and the inner diameter of the sampling needle is 0.6 mm.
In the step 2), the etching electrolyte is an aqueous solution of potassium bromide with the concentration of 1.5 mol/L, the flow velocity of the etching electrolyte is 10 mL/min, the etching voltage is 14 V, and the etching time is 1.5 min.
In the step 9), the temperature of heat treatment is 380 °C, and the time of heat treatment is 0.5 h.

### Embodiment 7

The difference from Embodiment 1 or 2 lies in that:
In the step 1), the material of the sampling needle is 430 ferritic stainless steel.
In the step 7), the mass concentration of the polytetrafluoroethylene (PTFE) emulsion is 50wt%, and the lifting speed is 150 mm/min.

### Embodiment 8

The difference from Embodiment 1 or 2 lies in that:
In the step 1), the material of the sampling needle is 420 martensitic stainless steel, and the inner diameter of the sampling needle is 0.9 mm.
In the step 5), the etching electrolyte is a water and glycerol solution of sodium chloride with the concentration of 0.5 mol/L (the volume ratio of water to glycerol is 1:1), the flow velocity of the etching electrolyte is 25 mL/min, the etching voltage is 40 V, and the etching time is 0.5 min.
In the step 7), the mass concentration of the polytetrafluoroethylene (PTFE) emulsion is 45wt%, and the lifting speed is 150 mm/min.

### Embodiment 9

The difference from Embodiment 1 or 2 lies in that:
In the step 1), the material of the sampling needle is 409 ferritic stainless steel.
In the step 5), the etching electrolyte is a glycerol solution of sodium chloride with the concentration of 0.15 mol/L, the flow velocity of the etching electrolyte is 25 mL/min, the etching voltage is 35 V, and the etching time is 10 min.
In the step 7), the mass concentration of the polytetrafluoroethylene (PTFE) emulsion is 55wt%, and the lifting speed is 80 mm/min.

The result of the embodiment shows that according to the present invention, firstly, a micro/nanoscale rough structure is constructed on the surface of the sampling needle by adopting a cathode-coated three-electrode dynamic coaxial etching method; then a polytetrafluoroethylene (PTFE) film is prepared on the substrate surface of the sampling needle with the micron rough structure by using a dip-coating film forming method. The inner surface, the outer surface and the end face of the prepared sampling needle are in a rolling superhydrophobic state, the prepared coating has good rub resistance, the contamination carrying possibility of the sampling needle can be reduced, the service life of the sampling needle is prolonged, and the method can be widely applied to the field of preparation of a superhydrophobic rub-resistant coating of a sampling needle.

## Claims

1. A method for preparing a rub-resistant superhydrophobic coating on the surface of a sampling needle, comprising the following steps:
1) cleaning and drying the sampling needle (1), a sealed cathode tube (2) and a cathode wire (3);
2) coating the surface of the cathode wire (3) with a high-molecular polymer solution and drying;
3) inserting the cathode wire (3) into the cathode tube (2), fixing the cathode wire (3) at the axis of the cathode tube (2) as a cathode, inserting the sampling needle (1) into the cathode tube (2), so that the cathode wire (3) is inserted into the sampling needle (2);
4) connecting a nozzle at one end of the cathode tube (2) to a dynamic solution supply device;
5) connecting the sampling needle (1) to a positive pole of a power supply (7), and connecting the cathode wire (3) and the cathode tube (2) to a negative pole of the power supply (7); and after power-on, carrying out electrochemical etching, while the dynamic solution supply device supplies an etching electrolyte into the cathode tube (2); thereby a micro/nanoscale rough structure is formed on the surface of the sampling needle (1);
6) after electrochemical etching is completed, cleaning and drying the sampling needle (1);
7) inserting the sampling needle (1) vertically into a polytetrafluoroethylene (PTFE) emulsion, holding for a certain period of time, and lifting the sampling needle (1) up; and
8) taking out and drying the sampling needle (1) to obtain the sampling needle (1) with a rub-resistant superhydrophobic coating on the surface.

2. The method for preparing a rub-resistant superhydrophobic coating on the surface of a sampling needle according to claim 1, wherein a post-treatment after the step 7 is: step 9) carrying out heat treatment on the sampling needle (1).

3. The method for preparing a rub-resistant superhydrophobic coating on the surface of a sampling needle according to claim 1, wherein
the material of the sampling needle (1) is a stainless steel material selected from ferritic stainless steel, austenitic stainless steel and martensitic stainless steel; the material of the cathode wire is a conductive material selected from stainless steel wire, copper wire, iron wire, platinum wire and titanium wire;
the material of the cathode tube (2) is a conductive materials selected from a metal tube, an alloy tube and a graphite tube; the material for sealing the cathode tube is consistent with the material of the cathode tube; the size of a central hole is kept consistent with the diameter of the cathode wire, two round holes with a diameter of 1.5 mm are formed along the central line at a position of 3 mm away from the central hole, to serve as solution inflow holes, and the cleaning time is 0.5-4 h.

4. The method for preparing a rub-resistant superhydrophobic coating on the surface of a sampling needle according to claim 1, wherein in the step 1), the sampling needle (1), the sealed cathode tube (2) and the cathode wire (3) are cleaned by ultrasonic oscillation in detergent, alcohol and deionised water successively, wherein the cleaning time is 0.5-4 h; then the sampling needle (1), the cathode tube (2) and the cathode wire (3) are blow-dried by using a nitrogen spray gun; and then the sampling needle (1), the cathode tube (2) and the cathode wire (3) are dried, wherein the drying temperature is 50-80 °C, and the drying time is 0.5-4 h.

5. The method for preparing a rub-resistant superhydrophobic coating on the surface of a sampling needle according to claim 1, wherein in the step 2), using the dried cathode wire (3) in the step 2) as a matrix, and repeatedly coating the surface of the cathode wire (3) with the high-molecular polymer solution.

6. The method for preparing a rub-resistant superhydrophobic coating on the surface of a sampling needle according to claim 1, wherein
in the step 4), the cathode tube (2) is vertically fixed, and a nozzle at the lower end of the cathode tube (2) is connected to the dynamic solution supply device.

7. The method for preparing a rub-resistant superhydrophobic coating on the surface of a sampling needle according to claim 1, wherein
in the step 5), the etching electrolyte is conveyed by a peristaltic pump (5); a solvent of a solution for electrochemical etching is one solvent selected from water, ethylene glycol and glycerol; a solute of the solution for electrochemical etching is a halogen compound such as sodium chloride, potassium chloride, calcium chloride, copper chloride, ferric chloride, sodium bromide, calcium bromide, potassium bromide, copper bromide, ferric bromide, and the concentration of the solution is 0.05-3 mol/L; the voltage of electrochemical etching is 5-40 V, and the time of electrochemical etching is 0.5-120 min.

8. The method for preparing a rub-resistant superhydrophobic coating on the surface of a sampling needle according to claim 1, wherein
in the step 7), the polytetrafluoroethylene (PTFE) emulsion is an emulsion formed by dispersing PTFE particles in water, the mass fraction of the polytetrafluoroethylene (PTFE) is 20-60%, the time period of immersing the sampling needle (1) into the emulsion is 2-60 s, and the lifting speed is 20-200 mm/min.

9. The method for preparing a rub-resistant superhydrophobic coating on the surface of a sampling needle according to claim 2, wherein the temperature of heat treatment is 360-380 °C, and the time period of heat treatment is 0.5-3 h.

## Patentansprüche

1. Verfahren zum Herstellen einer reibungsfesten superhydrophoben Beschichtung auf der Oberfläche einer Abtastnadel, umfassend die folgenden Schritte:
1) Reinigen und Trocknen der Abtastnadel (1), einer abgedichteten Kathodenröhre (2) und eines Kathodendrahtes (3);
2) Beschichten der Oberfläche des Kathodendrahtes (3) mit einer hochmolekularen Polymerlösung und Trocknen;
3) Einführen des Kathodendrahtes (3) in die Kathodenröhre (2), Fixieren des Kathodendrahtes (3) an der Achse der Kathodenröhre (2) als Kathode, Einführen der Abtastnadel (1) in die Kathodenröhre (2), sodass der Kathodendraht (3) in die Abtastnadel (2) eingeführt ist;
4) Verbinden einer Düse an einem Ende der Kathodenröhre (2) mit einer dynamischen Lösungszufuhrvorrichtung;
5) Verbinden der Abtastnadel (1) mit einem positiven Pol einer Leistungszufuhr (7) und Verbinden des Kathodendrahtes (3) und der Kathodenröhre (2) mit einem negativen Pol der Leistungszufuhr (7); und nach dem Einschalten Durchführen von elektrochemischem Ätzen, während die dynamische Lösungszufuhrvorrichtung einen Ätzelektrolyten in die Kathodenröhre (2) zuführt; wodurch eine mikro-/nanoskalige raue Struktur auf der Oberfläche der Abtastnadel (1) gebildet wird;
6) nachdem elektrochemisches Ätzen abgeschlossen ist, Reinigen und Trocknen der Abtastnadel (1);
7) Einführen der Abtastnadel (1) vertikal in eine Polytetrafluorethylen(PTFE-)Emulsion, Halten für einen bestimmten Zeitraum und Anheben der Abtastnadel (1); und
8) Herausnehmen und Trocknen der Abtastnadel (1), um die Abtastnadel (1) mit einer reibungsfesten superhydrophoben Beschichtung auf der Oberfläche zu erhalten.

2. Verfahren zum Herstellen einer reibungsfesten superhydrophoben Beschichtung auf der Oberfläche einer Abtastnadel nach Anspruch 1, wobei eine Nachbehandlung nach dem Schritt 7 wie folgt ist: Schritt 9) Durchführen von Wärmebehandlung an der Abtastnadel (1).

3. Verfahren zum Herstellen einer reibungsfesten superhydrophoben Beschichtung auf der Oberfläche einer Abtastnadel nach Anspruch 1, wobei das Material der Abtastnadel (1) ein Edelstahlmaterial ist, das aus ferritischem Edelstahl, austenitischem Edelstahl und martensitischem Edelstahl ausgewählt ist; das Material des Kathodendrahtes ein leitendes Material ist, das aus Edelstahldraht, Kupferdraht, Eisendraht, Platindraht und Titandraht ausgewählt ist; das Material der Kathodenröhre (2) ein leitendes Material ist, das aus einer Metallröhre, einer Legierungsröhre und einer Graphitröhre ausgewählt ist; das Material zum Abdichten der Kathodenröhre konsistent mit dem Material der Kathodenröhre ist; die Größe eines zentralen Lochs konsistent mit dem Durchmesser des Kathodendrahtes gehalten wird, zwei runde Löcher mit einem Durchmesser von 1,5 mm entlang der zentralen Linie an einer Position 3 mm weg von dem zentralen Loch gebildet sind, um als Lösungszuflusslöcher zu dienen, und die Reinigungszeit 0,5-4 h ist.

4. Verfahren zum Herstellen einer reibungsfesten superhydrophoben Beschichtung auf der Oberfläche einer Abtastnadel nach Anspruch 1, wobei in Schritt 1) die Abtastnadel (1), die abgedichtete Kathodenröhre (2) und der Kathodendraht (3) nacheinander durch Ultraschallschwingung in Reinigungsmittel, Alkohol und entionisiertem Wasser gereinigt werden, wobei die Reinigungszeit 0,5-4 h ist; dann die Abtastnadel (1), die Kathodenröhre (2) und der Kathodendraht (3) durch Verwenden einer Stickstoffsprühpistole blasgetrocknet werden; und dann die Abtastnadel (1), die Kathodenröhre (2) und der Kathodendraht (3) getrocknet werden, wobei die Trocknungstemperatur 50-80 °C ist und die Trocknungszeit 0,5-4 h ist.

5. Verfahren zum Herstellen einer reibungsfesten superhydrophoben Beschichtung auf der Oberfläche einer Abtastnadel nach Anspruch 1, wobei in dem Schritt 2) der getrocknete Kathodendraht (3) in dem Schritt 2) als Matrix verwendet wird und die Oberfläche des Kathodendrahtes (3) wiederholt mit der hochmolekularen Polymerlösung beschichtet wird.

6. Verfahren zum Herstellen einer reibungsfesten superhydrophoben Beschichtung auf der Oberfläche einer Abtastnadel nach Anspruch 1, wobei in dem Schritt 4) die Kathodenröhre (2) vertikal fixiert wird und eine Düse an dem unteren Ende der Kathodenröhre (2) mit der dynamischen Lösungszufuhrvorrichtung verbunden wird.

7. Verfahren zum Herstellen einer reibungsfesten superhydrophoben Beschichtung auf der Oberfläche einer Abtastnadel nach Anspruch 1, wobei in dem Schritt 5) der Ätzelektrolyt durch eine peristaltische Pumpe (5) befördert wird; ein Lösungsmittel einer Lösung für elektrochemisches Ätzen ein Lösungsmittel ausgewählt aus Wasser, Ethylenglykol und Glycerin ist; ein gelöster Stoff der Lösung für elektrochemisches Ätzen eine Halogenverbindung wie Natriumchlorid, Kaliumchlorid, Calciumchlorid, Kupferchlorid, Eisenchlorid, Natriumbromid, Calciumbromid, Kaliumbromid, Kupferbromid, Eisenbromid ist und die Konzentration der Lösung 0,05-3 mol/l ist; die Spannung des elektrochemischen Ätzens 5-40 V ist und die Zeit des elektrochemischen Ätzens 0,5-120 min ist.

8. Verfahren zum Herstellen einer reibungsfesten superhydrophoben Beschichtung auf der Oberfläche einer Abtastnadel nach Anspruch 1, wobei in dem Schritt 7) die Polytetrafluorethylen(PTFE-)Emulsion eine Emulsion ist, die durch Dispergieren von PTFE-Partikeln in Wasser gebildet wird, der Massenanteil des Polytetrafluorethylens (PTFE) 20-60 % ist, der Zeitraum des Eintauchens der Abtastnadel (1) in die Emulsion 2-60 s ist und die Hubgeschwindigkeit 20-200 mm/min ist.

9. Verfahren zum Herstellen einer reibungsfesten superhydrophoben Beschichtung auf der Oberfläche einer Abtastnadel nach Anspruch 2, wobei die Temperatur der Wärmebehandlung 360-380 °C ist und der Zeitraum der Wärmebehandlung 0,5-3 h ist.

## Revendications

1. Procédé permettant la préparation d'un revêtement superhydrophobe résistant au frottement sur la surface d'une aiguille d'échantillonnage, comprenant les étapes suivantes :
1) le nettoyage et le séchage de l'aiguille d'échantillonnage (1), d'un tube cathodique scellé (2) et d'un fil cathodique (3) ;
2) le revêtement de la surface du fil cathodique (3) avec une solution de polymère de poids moléculaire élevé et le séchage ;
3) l'insertion du fil cathodique (3) dans le tube cathodique (2), la fixation du fil cathodique (3) au niveau de l'axe du tube cathodique (2) en tant que cathode, l'insertion de l'aiguille d'échantillonnage (1) dans le tube cathodique (2), afin que le fil cathodique (3) soit inséré dans l'aiguille d'échantillonnage (2) ;
4) le raccordement d'une buse à une extrémité du tube cathodique (2) à un dispositif d'alimentation en solution dynamique ;
5) le raccordement de l'aiguille d'échantillonnage (1) à un pôle positif d'une alimentation électrique (7), et le raccordement du fil cathodique (3) et du tube cathodique (2) à un pôle négatif de l'alimentation électrique (7) ; et après la mise sous tension, la réalisation d'une gravure électrochimique, tandis que le dispositif d'alimentation en solution dynamique fournit un électrolyte de gravure dans le tube cathodique (2) ; ainsi, une structure rugueuse à l'échelle micro/nanométrique est formée sur la surface de l'aiguille d'échantillonnage (1) ;
6) une fois la gravure électrochimique terminée, le nettoyage et le séchage de l'aiguille d'échantillonnage (1) ;
7) l'insertion de l'aiguille d'échantillonnage (1) verticalement dans une émulsion de polytétrafluoroéthylène (PTFE), le maintien pendant une certaine période et le soulèvement de l'aiguille d'échantillonnage (1) ; et
8) le retrait et le séchage de l'aiguille d'échantillonnage (1) pour obtenir l'aiguille d'échantillonnage (1) avec un revêtement superhydrophobe résistant au frottement sur la surface.

2. Procédé permettant la préparation d'un revêtement superhydrophobe résistant au frottement sur la surface d'une aiguille d'échantillonnage selon la revendication 1, dans lequel un post-traitement après l'étape 7 est : l'étape 9) la réalisation d'un traitement thermique sur l'aiguille d'échantillonnage (1).

3. Procédé permettant la préparation d'un revêtement superhydrophobe résistant au frottement sur la surface d'une aiguille d'échantillonnage selon la revendication 1, dans lequel
le matériau de l'aiguille d'échantillonnage (1) est un matériau en acier inoxydable choisi parmi l'acier inoxydable ferritique, l'acier inoxydable austénitique et l'acier inoxydable martensitique ; le matériau du fil cathodique est un matériau conducteur choisi parmi un fil en acier inoxydable, un fil de cuivre, un fil de fer, un fil de platine et un fil de titane ;
le matériau du tube cathodique (2) est un matériau conducteur choisi parmi un tube métallique, un tube en alliage et un tube en graphite ; le matériau permettant de sceller le tube cathodique est conforme au matériau du tube cathodique ; la taille d'un trou central est maintenue conforme au diamètre du fil cathodique, deux trous ronds d'un diamètre de 1,5 mm sont formés le long de la ligne centrale à une position de 3 mm à l'écart du trou central, pour servir de trous d'entrée de solution, et le temps de nettoyage est de 0,5 à 4 h.

4. Procédé permettant la préparation d'un revêtement superhydrophobe résistant au frottement sur la surface d'une aiguille d'échantillonnage selon la revendication 1, dans lequel à l'étape 1), l'aiguille d'échantillonnage (1), le tube cathodique scellé (2) et le fil cathodique (3) sont nettoyés par oscillation ultrasonore dans du détergent, de l'alcool et de l'eau désionisée successivement, dans lequel le temps de nettoyage est de 0,5 à 4 h ; puis l'aiguille d'échantillonnage (1), le tube cathodique (2) et le fil cathodique (3) sont séchés par soufflage à l'aide d'un pistolet pulvérisateur d'azote ; et ensuite l'aiguille d'échantillonnage (1), le tube cathodique (2) et le fil cathodique (3) sont séchés, dans lequel la température de séchage est de 50 à 80 °C, et le temps de séchage est de 0,5 à 4 h.

5. Procédé permettant la préparation d'un revêtement superhydrophobe résistant au frottement sur la surface d'une aiguille d'échantillonnage selon la revendication 1, dans lequel, à l'étape 2), l'utilisation du fil cathodique séché (3) à l'étape 2) comme matrice, et le revêtement répété de la surface du fil cathodique (3) avec la solution de polymère de poids moléculaire élevé.

6. Procédé permettant la préparation d'un revêtement superhydrophobe résistant au frottement sur la surface d'une aiguille d'échantillonnage selon la revendication 1, dans lequel
à l'étape 4), le tube cathodique (2) est fixé verticalement, et une buse à l'extrémité inférieure du tube cathodique (2) est raccordée au dispositif d'alimentation en solution dynamique.

7. Procédé permettant la préparation d'un revêtement superhydrophobe résistant au frottement sur la surface d'une aiguille d'échantillonnage selon la revendication 1, dans lequel
dans l'étape 5), l'électrolyte de gravure est transporté par une pompe péristaltique (5) ; un solvant d'une solution pour la gravure électrochimique est un solvant choisi parmi l'eau, l'éthylène glycol et le glycérol ; un soluté de la solution pour la gravure électrochimique est un composé halogéné tel que le chlorure de sodium, le chlorure de potassium, le chlorure de calcium, le chlorure de cuivre, le chlorure ferrique, le bromure de sodium, le bromure de calcium, le bromure de potassium, le bromure de cuivre, le bromure ferrique, et la concentration de la solution est de 0,05 à 3 mol/l ; la tension de la gravure électrochimique est de 5 à 40 V, et le temps de la gravure électrochimique est de 0,5 à 120 min.

8. Procédé permettant la préparation d'un revêtement superhydrophobe résistant au frottement sur la surface d'une aiguille d'échantillonnage selon la revendication 1, dans lequel
à l'étape 7), l'émulsion de polytétrafluoroéthylène (PTFE) est une émulsion formée par dispersion de particules de PTFE dans de l'eau, la fraction massique du polytétrafluoroéthylène (PTFE) est de 20 à 60 %, la période d'immersion de l'aiguille d'échantillonnage (1) dans l'émulsion est de 2 à 60 s, et la vitesse de levage est de 20 à 200 mm/min.

9. Procédé permettant la préparation d'un revêtement superhydrophobe résistant au frottement sur la surface d'une aiguille d'échantillonnage selon la revendication 2, dans lequel la température de traitement thermique est de 360 à 380 °C, et la période de traitement thermique est de 0,5 à 3 h.
